# EUROPEAN PATENT SPECIFICATION

(11) **EP 1 644 331 B1**
(45) Date of publication and mention of the grant of the patent: **20.02.2008**
(21) Application number: 04743017.8
(22) Date of filing: 21.06.2004
(51) Int. Cl.: C07D 213/16

(54) **A METHOD FOR PRODUCING 1,3-DIALKYLPYRIDINIUM OLIGOMERS AND RELATED COMPOUNDS USING A SOLID SUPPORT**
EINE FESTPHASENGEBUNDENE METHODE ZUR HERSTELLUNG VON 1,3-DIALKYLPYRIDINIUM OLIGOMEREN UND VERWANDTEN VERBINDUNGEN
METHODE POUR PRODUIRE DES OLIGOMERES 1,3-DIALKYLPYRIDINIUM ET DES COMPOSES ASSOCIES FAISANT APPEL A UN SUPPORT SOLIDE

(30) Priority: 19.06.2003 GB 0314341
(43) Date of publication of application: 12.04.2006
(73) Proprietor: ABERDEEN UNIVERSITY, Aberdeen AB24 3FX (GB)
(72) Inventor: JASPARS, Marcel, Old Aberdeen AB24 3UE (GB)
(74) Representative: Peebles, Katrina
(86) International application number: PCT/GB2004/002666
(87) International publication number: WO 2004/113299

(56) References cited:
- WO-A-03/040094
- MICHELLIZA ET AL: "Synthesis of the Cytotoxic Sponge Metabolite Haliclamine A" JOURNAL OF ORGANIC CHEMISTRY, vol. 67, 2002, pages 6474-6478, XP002300394
- WANNER ET AL: "Synthesis of the Cyclostellatamines A-F and Related Bis(3-alkylpyridinium) Macrocycles" EUROPEAN JOURNAL OF ORGANIC CHEMISTRY, 1998, pages 889-895, XP002300395
- PETRICCI ET AL: "An improved Synthesis of Solid-Supported Reagents (SSRs) for selective Acylation of Amines by Microwave Irradiation" TETRAHEDRON LETTERS, vol. 43, 2002, pages 6507-6509, XP002300397

## Description

The present invention relates to a method for producing a di-substituted pyridinium compound.

Dialkylpyridinium compounds, (Di-APS), in particular 1,3-APS oligomers, are known to be produced by sponges, for example the Haploscerid genera such as *Haliclona, Amphimedon and Callyspongia*, as part of their chemical defences, and have potentially useful biological properties. Diverse biological activities have been identified for different 1,3-APS compositions, including cytotoxicity, neurotoxicity and inhibition of action potentials, stimulation of transmitter release, inhibition of K⁺ conductances, and anticholinesterase activity. At least some of these observed actions of 1,3-APS compositions relate to the pore forming or membrane lesion effects of these compounds, which properties may be useful, for example, in the transfection of cells with genetic material.

Naturally occurring 1,3-APS compounds are produced as cocktails of different 1,3-APS compounds, from which individual 1,3-APS compounds are difficult to isolate, due to the same basic structure and very similar molecular weights of the different 1,3-APS compounds. However, it is desirable to be able to isolate different individual 1,3-APS compounds, in order to determine their different biological activities, for example with regard to the effect of degree of polymerisation, and length and rigidity of linking chains.

An alternative approach to isolating individual naturally occurring Di-APS compounds, is to synthesise these compounds by laboratory methods. Di-APS compounds generally occur as high molecular weight linear oligomers, having a molecular weight ranging from 1 KDa to greater than 25 KDa, having varying lengths of aliphatic chains linking the pyridine units. However, model linear Di-APS compounds closely analogous to the natural products remain to be synthesized by laboratory methods in a controllable fashion.

Previous studies have succeeded in producing linear 1,3-APS oligomers, but the methods employed meant that the product obtained consisted of a mixture of linear and cyclic oligomers with a wide range of molecular weights (see for example, Davies-Coleman, M.T.; Faulkner, D.J. J. Org. Chem. 1993, 58, 5925-5930). The approach used in these studies was to synthesize the 3-substituted pyridine monomer and introduce a good leaving group, for example bromide, at the end of the alkyl chain. This monomer was then refluxed to initiate polymerisation. Alternative methods based on this strategy incorporate an ether linkage in the linking chain (Gil, L. et al Tetrahedron Lett. 1995, 36, 2059-2062), or form cyclic dimers (Morimoto, Y.; Yokoe, C. Tetrahedron Lett. 1997, 38, 8981-8984)or tetramers with short linking chains(Shinoda, S. et al, Chem. Commun. 1998, 181).

A further study for producing macrocyclic 1,3-APS compounds is disclosed in Kaiser, A. et al., J. Am. Chem. Soc. 1998, 120, 8026-8034, which involves the formation and subsequent reaction of an N-(2,4-dinitrobenzene) pyridinium salt (Zinke salt) to yield linear 1,3-APS compounds. However, this method has certain disadvantages, in that it is relatively complex, and has only been shown to work for small macrocyclic oligomers.

Accordingly, there still exists a need for a reliable method for synthesizing linear di-substituted pyridinium compounds, for example di-APS oligomers and polymers. The present invention seeks to provide such a method, which overcomes the aforementioned disadvantages of previous methods.

According to the present invention there is provided a method of producing a linear di-substituted pyridinium compound, the method comprising the steps of:
(a) attaching a first 2, 3, or 4-substituted pyridine compound of the formula NC₅R₄-R'-X (1) to a solid support, to form a compound of the formula NC₅R₄-R'-Y-SUPPORT (2), wherein SUPPORT represents the solid support, R is selected from hydrogen, hydroxyl, and substituted or unsubstituted alkyl, alkoxy, aryl, alkaryl, aralkyl, and alkenyl groups, R' is a first linking group, X is a group which can react with the solid support to attach the first pyridine compound to the support, and Y is absent or is a second linking group;
(b) forming a di-substituted pyridine compound of the formula ⁻A-⁺NC₅R₄-R'-Z (3) from a second 2, 3, or 4-substituted pyridine compound of formula (1), which second pyridine compound may be the same as or different to the first pyridine compound, wherein A is a protecting group, and Z is a leaving group;
(c) reacting the compound of formula (2) formed in step (a) with the compound of formula (3) formed in step (b), to form a di-substituted pyridinium compound of the formula ⁻A-⁺NC₅R₄-R'-[⁻Q⁺NC₅R₄-R'-]ₙY-SUPPORT (4), wherein Q- is a counter ion and n=1;
(d) optionally, repeating step (c) as many times as required to obtain a compound of formula (4) wherein n is an integer of 2 or greater; and
(e) detaching the compound of formula (4) from the solid support, and removing the protecting group A to form a di-substituted pyridinium compound of the formula NC₅R₄-R'-[⁻Q⁺NC₅R₄-R'-]ₙ-X (5), wherein n is an integer, and Q⁻ and X are a counter ion and a group which can react with the solid support to attach the first pyridine compound to the support respectively, which may the same or different as Q and X defined above in steps (c) and (a) respectively.

Thus, during steps (a) to (d) of the method of the present invention, one end of the compound which is to become the product compound of formula (5) is bound to a solid support, thereby removing it from the reaction equilibrium, and substantially eliminating cyclisation. The use of protecting and leaving groups per the method of the present invention allows for the controlled addition of monomer units without macrocycle formation occurring in solution. The method of the present invention can be used to produce di-substituted pyridinium compounds in high rates of conversion, and in high yields. The method of the present invention also has the advantage that a variety of linking groups may be used, which may incorporate different functionalities, for example functionalities which can restrict the conformational space accessible by the di-substituted pyridinium compound, such as a double bond or cyclopropyl ring, or groups which allow the attachment of fluorescent labels, so that the di-substituted pyridinium compound can be localised intracellularly and in cell membranes.

Herein the term "di-substituted pyridinium compound" can refer to either a 1,2-, 1,3-, or 1,4-substituted pyridinium compound. However, the preferred products of the method of the present invention are 1,3-substituted pyridinium compounds, in particular linked 1,3-dialkyl pyridinium compounds.

Although the method of the present invention is suitable for producing oligomers and polymers potentially having a wide range of degrees of polymerisation, and hence molecular weights, for example n=1 to 20 in formula (5) above, it is particularly useful for producing oligomers having higher degrees of polymerisation, for example n=20 to 100 in formula (5) above.

Referring to the steps of the method of the present invention in turn, in step (a) a first 2,3,or 4-substituted, preferably 3-substituted, pyridine compound of the formula NC₅R₄-R'-X (1) is attached to a solid support, to form a compound of the formula NC₅R₄-R'-Y-SUPPORT (2), wherein SUPPORT represents the solid support, R is selected from hydrogen, and substituted or unsubstituted alkyl, aryl, alkaryl, aralkyl, and alkenyl groups, R' is a first linking group, X is a group which can react with the solid support to attach the first pyridine compound to the support, and Y is absent or is a second linking group. Each R group is preferably a hydrogen atom.

Compounds of formula (1) are commercially available, or may be synthesised as is known in the art. Thus, for example, a compound of formula (1) may be synthesised by reaction of a compound of formula Z'-R"-X with a suitable pyridine compound, with protection of the X- group as necessary, wherein R" is a linking group and Z' is a suitable leaving group. For example, a compound of formula (1) may be prepared by reacting Br-R"-OH (i.e. Z'=Br and X=OH) with t-butyldimethyl-chlorosilane (TBDMSC1) to form Br-R"-OTBDMS, which may be reacted with 2,3,or 4-methylpyridine (2,3, or 4-picoline) with deprotection of the X group to form NC₅H₄-R-OH, wherein R' is as defined above, i.e. a linking group equivalent to R" plus one carbon atom. Of course, protecting groups other than TBDMSCl may be used, as will be apparent to those skilled in the art.

As referred to above, the method of the present invention is advantageous in that a variety of linking groups may be used, which may incorporate different functionalities. Preferred R' groups have no terminal carbon atoms, which further restricts the possibility of cyclisation occurring.

Thus, in formulas (1) to (5) above, each group R' may be the same or different and selected from an alkylene group (for example, a group -(CH₂)ₘ-, wherein m is an integer from 2 to 12, preferably from 6 to 10), an alkenyl-containing group (for example, a group having from 2 to 12 carbon atoms, preferably from 6 to 10 carbon atoms, containing one or more alkenyl groups, e.g. cis- or trans- 2-butenyl, 3-hexenyl, 2,5-hepten-di-yl, and 4-octenyl groups), a cyclopropanyl-containing group (for example, cis- or trans- -(CH₂)ₚ-cyclopropanyl-(CH₂)_{q}- wherein p and q are the same or different and are integers from 1 to 4, preferably 1 or 2). As discussed above, R' may also be a group which comprises a fluorescent label, so that the di-substituted pyridinium compound can be localised intracellularly and in cell membranes (for example, a linking group R' having a pendant alcohol group, for attachment of a fluorescent group).

Where appropriate, cis- or trans- isomerism may be introduced into linking group R' as is known in the art (for example, Morimoto, Y.; Yokoe, C. Tetrahedron Lett. 1997, 38, 8981-8984). A double bond may be converted into a cyclopropyl ring using carbene chemistry.

In the compound of formula (1), X is a group which can react with the solid support to attach the compound of formula (1) to the support. Thus, the particular group X to be used in a particular compound of formula (1) will depend upon the particular solid support being used. Thus, suitable X groups include hydroxyl, carboxyl, thiol, and amine groups. However, X is preferably a hydroxyl group, and particularly preferred compounds of formula (1) are pyridines 3-substituted by a hydroxyalkyl group, i.e. compounds of the formula NC₅R₄-(CH₂)ₙ-OH, wherein n is as defined above.

The solid support used in the present invention may be any suitable support to which the compound of formula (1) may be attached. Preferred solid support materials are organic resins having functionality which can react with group X of the compound of formula (1), described above. Particularly preferred solid support materials are trityl chloride and functionalised polystyrene resins (for example, Merryfield resins, i.e. chloromethylstyrene-divinylbenzene resins).

Group Y in formula (2) is absent or a second linking group, depending on the particular reaction which occurs between the compound of formula (1) and the solid support to form the compound of formula (2). Thus, group X in formula (1) may be eliminated, or, for example, in the case of a compound of formula (1) in which X is a hydroxyl group, the compound of formula (2) may have the more specific formula NC₅R₄-R'-O-SUPPORT, i.e. Y may be an oxygen atom.

In step (b) of the method of the present invention, a disubstituted pyridine compound of the formula ⁻A-⁺NC₅R₄-R'-Z (3) is formed from a second 2,3 or 4-substituted pyridine compound of formula (1), which second pyridine compound may be the same as or different to the first pyridine compound, wherein A is a protecting group, and Z is a leaving group. Step (b) is independent of step (a), and accordingly may be performed before, after, or simultaneously with step (a).

Step (b) itself thus includes two steps: a first step of converting group X to a leaving group Z, for reaction with the nitrogen atom of the pyridine group of the compound of formula (2), and a second step of protecting the nitrogen atom of the second pyridine compound, with a protecting group A.

In step (b), group X may be converted to a suitable leaving group Z as is known in the art. For example, as a hydroxyl group, X may be converted to a mesyl (methanesulphonyl) group by reaction with mesyl chloride. Other suitable leaving groups will be apparent to those skilled in the art.

The nitrogen atom of the second pyridine compound of formula (1) used in step (b) may be protected by convertion to the N-oxide as is known in the art, i.e. protecting group A is oxygen, for example by reaction of the nitrogen atom of the pyridine group with a peracid, for example m-chloroperbenzoic acid. Alternatively, the nitrogen atom may be protected by formation of the borane, i.e. A is BH₃-.

In step (c) of the method of the present invention, the compound of formula (2) formed in step (a) is reacted with the compound of formula (3) formed in step (b), to form a di-substituted pyridinium compound of the formula ⁻A-⁺NC₅R₄-R'-[⁻Q⁺NC₅R₄-R'-]ₙY-SUPPORT (4), wherein Q- is a counter ion and n=1. Thus, in step (c) two pyridine-containing monomer units are combined to form a dimeric di-substituted pyridinium compound, the leaving group Z of the compound of formula (3) reacting with the nitrogen atom of the pyridine group of the compound of formula (2). The reaction may take place on heating, in the presence of a suitable counter ion Q⁻, for example a chloride, iodide, or other suitable counter ion.

In step (d) of the method of the present invention, step (c) is optionally repeated as many times as required to obtain a compound of formula (4) wherein n is an integer of 2 or greater. If the desired di-substituted pyridinium compound end product is a dimer (i.e. a compound of formula (5) in which n=1), then no repetitions of step (c) are required. However, as discussed above, the method of the present invention is particularly useful for producing oligomers having higher degrees of polymerisation, for example n=20 to 100 in formula (5) above, which will of course require the repetition of step (c) the appropriate number of times.

In preferred embodiments of the present invention, chain extension to produce oligomers having a higher degree of polymerisation may be accelerated by releasing oligomers having a lower degree of polymerisation from the solid support as compounds having the formula (5), and reintroducing them as reagents as an alternative to, or in addition to, the second pyridine compound used in step (b). Thus, in these preferred embodiments, a compound of formula NC₅R₄-R'-[⁻Q⁺NC₅R₄-R'-]ₙ-X (5) may be converted to a compound of formula A⁻-⁺NC₅R₄-R'-[⁻Q⁺NC₅R₄-R'-]ₙ-Z (5a) per step (b), and the compound of formula (5a) may then be reacted with the compound of formula (2) formed in step (a) or (c), per step (d).

In step (e) of the method of the present invention, the compound of formula (4) is detached from the solid support, and reduced to form a di-substituted pyridinium compound of the formula NC₅R₄-R'-[⁻Q⁺NC₅R₄-R'-]ₙ-X (5), wherein n is an integer, and Q- and X are a counter ion and a group which can react with the solid support to attach the first pyridine compound to the support respectively. Q⁻ and X of step (e) may be the same or different as Q- and X defined above with reference steps (a) and (c) respectively. The compound of formula (4) may be detached from the solid support according to the particular solid support being used. Thus, in the case of a functionalised trityl chloride resin solid support, the compound of formula (4) may be detached using a strong acid. For example, if the strong acid used to detach the formula (4) from the solid support is hydrochloric acid, then counter ion Q- will be chloride. The protecting group A can be removed as is known in the art, for example by reducing the N-oxide to a nitrogen atom where A in formula (4) is oxygen.

An embodiment of the present invention will now be described in detail.

Reaction Scheme 1 below describes a preferred method for producing a preferred linear 1,3-alkylpyridinium compound of the present invention. 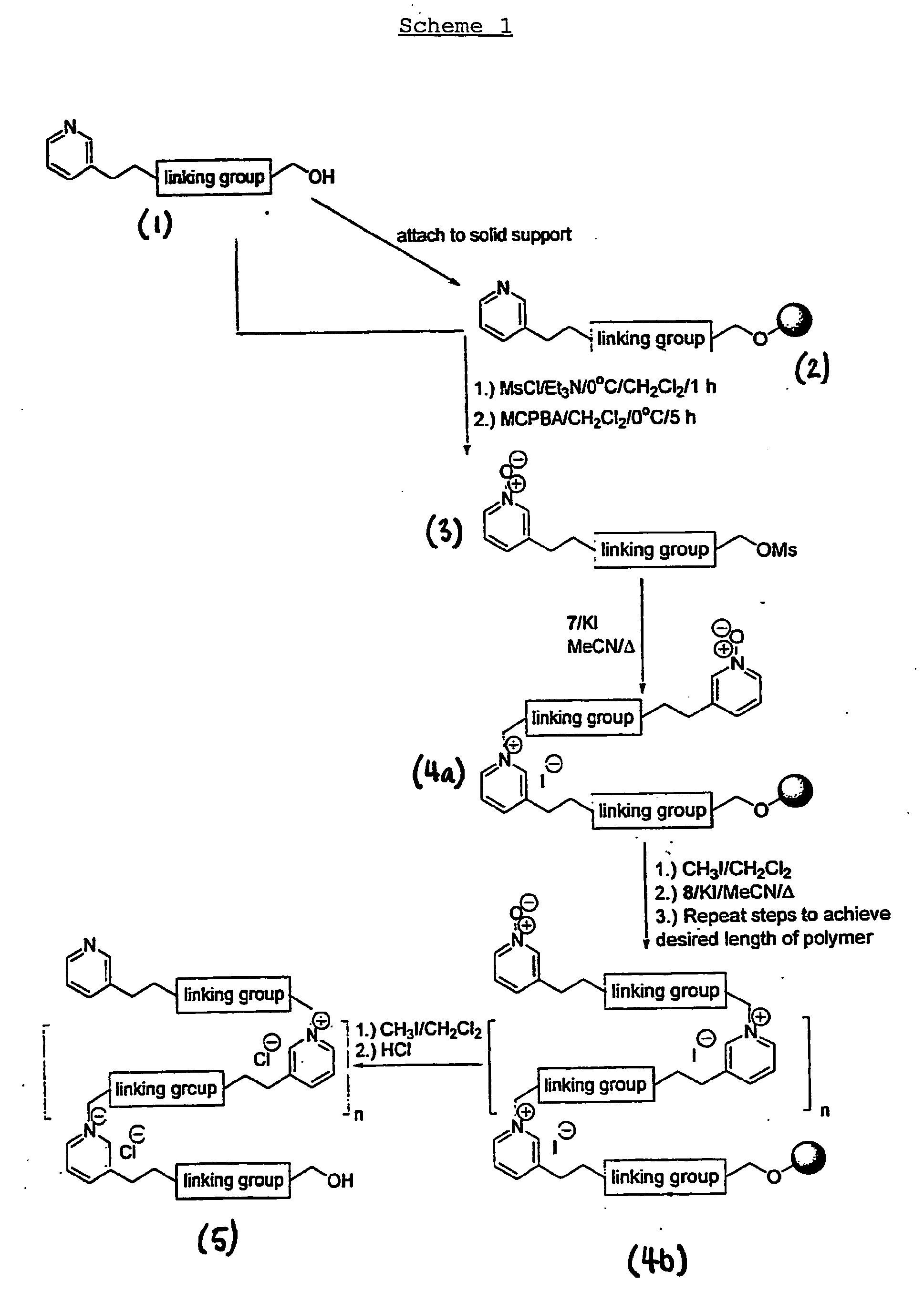 wherein: Ms=methanesulphonyl (mesyl)
Et=ethyl
MCPBA = m-chloroperbenzoic acid

Referring to Scheme 1, 3-substituted pyridine compound (1) is formed by firstly reacting Br-R"-OH with t-butyldimethylchlorosilane (TBDMSC1) to form Br-R"-OTBDMS, wherein R" is a suitable linking group (described hereinabove). The Br-R"-OTBDMS is then reacted with 3-methylpyridine (3-picoline) with deprotection of the X group to form NC₅R₄-R'-OH (3-substituted pyridine compound (1)), wherein R' is a linking group equivalent to R" plus one carbon atom.

A first molecule of 3-substituted pyridine compound (1) is then attached to a trityl chloride resin solid support, to form compound (2).

Before, after, or simultaneously with the formation of compound (2), a second molecule of 3-substituted pyridine compound (1) is reacted firstly with mesyl chloride, and secondly with m-chloroperbenzoic acid to form compound (3) of formula ⁻O-⁺NC₅R₄-R'-O-Ms, wherein Ms is a mesyl group.

Next, compound (2) is reacted with one equivalent of compound (3) to form 1,3-alkylpyridinium compound (4a) having the formula ⁻O-⁺NC₅R₄-R'-⁻I⁺NC₅R₄-R'-O-SUPPORT, i.e. two pyridine-containing monomer units are combined to form a dimeric 1,3-alkylpyridinium compound, the -Ms leaving group of compound (3) reacting with the nitrogen atom of the pyridine group of compound (2). This step is repeated the appropriate number of times to obtain an 1,3-alkylpyridinium compound end product having the desired degree of polymerisation, i.e. compound (2) is reacted with 1,3-alkylpyridinium compound (4a) to produce compound (4b) of formula ⁻O-⁺NC₅R₄-R'-[⁻I⁺NC₅R₄-R'-]ₙO-SUPPORT, wherein n is preferably 20 to 100.

As discussed above, in preferred embodiments of the present invention, chain extension to produce oligomers having a higher degree of polymerisation may be accelerated by releasing oligomers having a lower degree of polymerisation from the solid support as compounds having the formula (5), and reintroducing them as reagents as an alternative to, or in addition to, the second pyridine compound used in step (b). Thus, in these preferred embodiments, a compound of formula NC₅R₄-R'-[⁻Q⁺NC₅R₄-R'-]ₙ-X (5) may be converted to a compound of formula O⁻-⁺NC₅R₄-R'-[⁻Q⁺NC₅R₄-R'-]ₙ-Z (5a) per step (b), and the compound of formula (5a) may then be reacted with the compound of formula (2) formed in step (a) or (c), per step (d).

Finally in Scheme 1, compound (4b) is detached from the solid support, and reduced to form 1,3-alkylpyridinium compound (5) of formula NC₅R₄-R'-[⁻Cl⁺NC₅R₄-R'-]ₙ-OH using hydrochloric acid, as shown. The counter ion in final product (5) is thus chloride.

As referred to above, the method of the present invention is advantageous in that a variety of linker groups may be used, which may incorporate different functionalities.

Reaction Scheme 2 below describes a method for producing a compound from which starting material NC₅R₄-R'-X used in step (a) of the present invention may in turn be produced, in which R' is an alkenyl-containing linking group having either cis- or trans- isomerism. 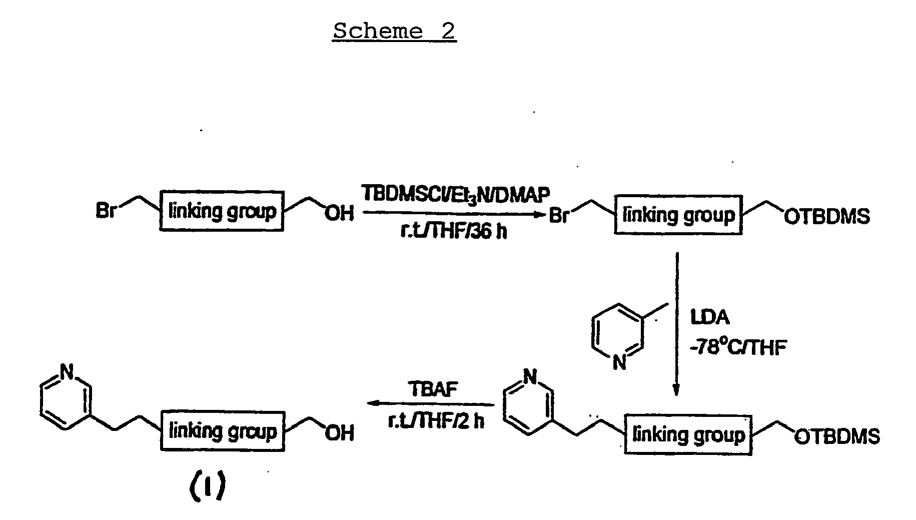 wherein: TBDMS=t-butyldimethylsilyl
DMAP=-4-dimethylaminopyridine
THF=tetrahydrofuran
LDA=lithium diisopropylamine
TBAF=t-butylammonium fluoride

Reaction Scheme 3 below describes a method for producing a compound from which starting material NC₅R₄-R'-X of formula (1) used in step (a) of the present invention may in turn be produced, in which R' is an alkylene linking group having a pendant alcohol group to which a fluorescent group may be attached. 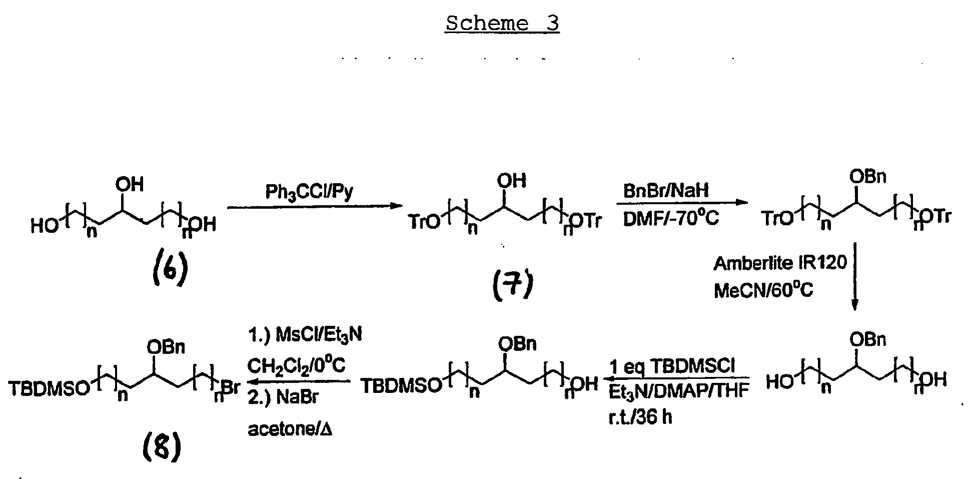 wherein: Ph=phenyl
Py=pyridine
Tr=trityl
Bn=benzyl

As shown in Scheme 3, triol (6) is differentially protected to form alcohol (7), which is subsequently converted to bromide (8), which can then in turn be used to form starting material NC₅R₄-R'-X of formula (1) used in step (a) of the present invention.

Fluorescent labels, available commercially, can be reacted with the monomer or oligomer, depending on the reaction conditions necessary. To avoid cell damage, the excitation wavelength of the fluorescent label is preferably above 350 nm, and the emission wavelength should preferably be greater than 80 nm higher, to permit ratiometric measurements. Suitable fluorescent labels include fluorescein, lissamine, and rhodamine based compounds, although other fluorescent materials may be used according to conditions. Such a linking group having a fluorescent group would preferably be incorporated into the 1,3-APS compound end product only once or twice.

## Claims

1. A method of producing a linear di-substituted pyridinium compound, the method comprising the steps of:
(a) attaching a first 2, 3, or 4-substituted pyridine compound of the formula NC₅R₄-R'-X (1) to a solid support, to form a compound of the formula NC₅R₄-R'-Y-SUPPORT (2), wherein SUPPORT represents the solid support, R is selected from hydrogen, hydroxyl, and substituted or unsubstituted alkyl, alkoxy, aryl, alkaryl, aralkyl, and alkenyl groups, R' is a first linking group, X is a group which can react with the solid support to attach the first pyridine compound to the support, and Y is absent or is a second linking group;
(b) forming a di-substituted pyridine compound of the formula ⁻A-⁺NC₅R₄-R'-Z (3) from a second 2, 3, or 4-substituted pyridine compound of formula (1), which second pyridine compound may be the same as or different to the first pyridine compound, wherein A is a protecting group, and Z is a leaving group;
(c) reacting the compound of formula (2) formed in step (a) with the compound of formula (3) formed in step (b), to form a di-substituted pyridinium compound of the formula ⁻A-⁺NC₅R₄-R'-[⁻Q⁺NC₅R₄-R'-]ₙY-SUPPORT (4), wherein Q⁻ is a counter ion and n=1;
(d) optionally, repeating step (c) as many times as required to obtain a compound of formula (4) wherein n is an integer of 2 or greater; and
(e) detaching the compound of formula (4) from the solid support, and reducing to form a di-substituted pyridinium compound of the formula NC₅R₄-R'-[⁻Q⁺NC₅R₄-R'-]ₙ-X (5), wherein n is an integer, and Q- and X are a counter ion and a group which can react with the solid support to attach the first pyridine compound to the support respectively, which may the same or different as Q and X defined above in steps (c) and (a) respectively.

2. A method according to claim 1 wherein each R group is hydrogen.

3. A method according to claim 1 or 2 wherein in step (d), step (c) is repeated such that in formula (5) n=20 to 100.

4. A method according to claim 1, 2 or 3 wherein the compound of formula (1) is prepared by reaction of a compound of formula Z'-R"-X with a pyridine compound, with protection of the X- group as necessary, wherein R" is a linker group and Z' is a suitable leaving group.

5. A method according to claim 4 wherein the compound of formula (1) is prepared by reacting Br-R"-OH with t-butyldimethyl-chlorosilane (TBDMSC1) to form Br-R"-OTBDMS, which is reacted with 2,3 or 4-methylpyridine (2,3, or 4-picoline) with deprotection of the X group to form NC₅R₄-R'-OH.

6. A method according to any one of claims 1 to 5 wherein linker group R' has no terminal carbon atoms.

7. A method according to any preceding claim wherein each group R' is the same or different and is selected from an alkylene group, an alkenyl-containing group, and a cyclopropanyl-containing group.

8. A method according to claim 7 wherein each group R' is the same or different and is selected from a group -(CH₂)ₘ-, wherein m is an integer from 2 to 12, a group having from 2 to 12 carbon atoms containing one or more alkenyl groups, and cis- or trans- -(CH₂)ₚ-cyclopropanyl-(CH₂)_{q}- wherein p and q are the same or different and are integers from 1 to 4.

9. A method according to any preceding claim wherein R' is a group which comprises a fluorescent group, or a group to which a fluorescent group can be attached.

10. A method according to claim 9 wherein R' has a pendant alcohol group, for attachment of a fluorescent group.

11. A method according to any preceding claim wherein in formula (1) X is selected from hydroxyl, carboxyl, thiol, and amine groups.

12. A method according to claim 11 wherein X is a hydroxyl group.

13. A method according to claim 12 wherein the compound of formula (1) is a compound of the formula NC₅R₄-(CH₂)ₙ-OH.

14. A method according to any preceding claim wherein the solid support material comprises an organic resin having functionality which can react with group X of the compound of formula (1).

15. A method according to claim 14 wherein the solid support material comprises trityl chloride or a functionalised polystyrene resin.

16. A method according to any preceding claim wherein group Y in formula (2) is an oxygen atom.

17. A method according to any preceding claim wherein in step (b) group X is converted to a mesyl (methanesulphonyl) group by reaction with mesyl chloride.

18. A method according to any preceding claim wherein A is oxygen or BH₃- .

19. A method according to claim 18 wherein A is oxygen, and the nitrogen atom of the second pyridine compound of formula (1) used in step (b) is converted to the N-oxide by reaction of the nitrogen atom of the pyridine group with a peracid.

20. A method according to claim 19 wherein the peracid comprises m-chloroperbenzoic acid.

21. A method according to any preceding claim wherein counter ion Q- in step (c) is an iodide ion.

22. A method according to any preceding claim wherein oligomers having the formula (5) are released from the solid support and reintroduced as reagents as an alternative to, or in addition to, the second pyridine compound used in step (b).

23. A method according to claim 22 wherein a compound of formula NC₅R₄-R'-[⁻Q⁺NC₅R₄-R'-]ₙ-X (5) is converted to a compound of formula ⁻A-⁺NC₅R₄-R'-[⁻Q⁺NC₅R₄-R'-]ₙ-Z (5a) per step (b), and the compound of formula (5a) is then reacted with the compound of formula (2) formed in step (a) or (c), per step (d).

24. A method according to any preceding claim wherein the compound of formula (4) is detached from the solid support, and reduced to form a di-substituted pyridinium compound of the formula NC₅R₄-R'-[⁻Q⁺NC₅R₄-R'-]ₙ-X (5) using an acid.

25. A method according to claim 24 wherein the acid is hydrochloric acid, and counter ion Q⁻ is chloride.

26. A method according to any preceding claim wherein the di-substituted di-substituted pyridinium compound is a linked dialkyl pyridinium compound.

## Patentansprüche

1. Verfahren zur Herstellung einer linearen zweifach substituierten Pyridiniumverbindung, das Verfahren umfaßt die Schritte:
(a) Anlagern einer ersten 2-, 3- oder 4-substituierten Pyridinverbindung der Formel NC₅R₄-R'-X (1) an einen festen Träger, um eine Verbindung der Formel NC₅R₄-R'-Y-TRÄGER (2) zu bilden, wobei der TRÄGER einen festen Träger verkörpert, R ist ausgewählt aus Wasserstoff, Hydroxyl- und substituierten und nicht substituierten Alkyl-, Alkoxy-, Aryl-, Alkaryl-, Aralkyl- und Alkenylgruppen, R' ist eine erste Verknüpfungsgruppe, X ist eine Gruppe, die mit dem festen Träger reagieren kann, um die erste Pyridinverbindung an den Träger anzulagern, Y fehlt oder ist eine zweite Verknüpfungsgruppe;
(b) Bilden einer zweifach substituierten Pyridinverbindung der Formel ⁻A-⁺NC₅R₄-R'-Z (3) aus einer zweiten 2-, 3- oder 4-substituierten Pyridinverbindung der Formel (1), wobei die zweite Pyridinverbindung gleich wie oder verschieden zu der ersten Pyridinverbindung sein kann, wobei A eine Schutzgruppe und Z eine austretende Gruppe ist;
(c) Umsetzen der Verbindung der Formel (2), die in Schritt (a) hergestellt wurde, mit der Verbindung der Formel (3), die in Schritt (b) gebildet wurde, um eine zweifach substituierte Pyridiniumverbindung der Formel ⁻A-⁺NC₅R₄-R'-[⁻Q⁺NC₅R₄-R'-]ₙY-TRÄGER (4) herzustellen, wobei Q⁻ ein Gegenion und n=1 ist;
(d) wahlweise Wiederholen von Schritt (c), so oft wie nötig, um eine Verbindung der Formel (4) zu erhalten, wobei n eine ganze Zahl von 2 oder mehr ist; und
(e) Ablösen der Verbindung der Formel (4) von dem festen Träger und Reduzieren, um eine zweifach substituierte Pyridiniumverbindung der Formel NC₅R₄-R'-[⁻Q⁺NC₅R₄-R'-]ₙ-X (5) zu bilden, wobei n eine ganze Zahl ist, Q⁻ und X sind Gegenionen und eine Gruppe, die mit dem festen Träger reagieren kann, um die erste Pyridinverbindung entsprechend an den Träger anzufügen, die gleich oder verschieden sein können wie Q und X, die oben in den Schritten (c) beziehungsweise (a) definiert sind.

2. Verfahren nach Anspruch 1, wobei jede R-Gruppe Wasserstoff ist.

3. Verfahren nach Anspruch 1 oder 2, wobei in Schritt (d), Schritt (c) wiederholt wird, so daß in Formel (5) n=20 bis 100 ist.

4. Verfahren nach Anspruch 1, 2 oder 3, wobei die Verbindung der Formel (1) hergestellt wird durch Umsetzung einer Verbindung Z'-R"-X mit einer Pyridinverbindung mit Schutz der X-Gruppe, wenn nötig, wobei R" eine Verknüpfungsgruppe und Z' eine geeignete austretende Gruppe ist.

5. Verfahren nach Anspruch 4, bei dem die Verbindung der Formel (1) hergestellt wird durch Umsetzung von Br-R"-OH mit t-Butyldimethyl-chlorsilan (TBDMSCI), um Br-R"-OTBDMS zu bilden, das mit 2-, 3- oder 4-Methylpyridin (2-, 3- oder 4-Picolin) mit Entfernung des Schutzes der X-Gruppe umgesetzt wird, um NC₅R₄-R'-OH zu bilden.

6. Verfahren nach einen der Ansprüche 1 bis 5, wobei die Verknüpfungsgruppe R' keine terminalen Kohlenstoffatome hat.

7. Verfahren nach einem der vorherigen Ansprüche, wobei jede R'-Gruppe gleich oder verschieden ist und ausgewählt wird aus einer Alkylengruppe, einer Alkylen-enthaltenden Gruppe und einer Cyclopropanyl-enthaltenden Gruppe.

8. Verfahren nach Anspruch 8, bei dem jede R'-Gruppe gleich oder verschieden ist und ausgewählt wird aus einer -(CH₂)ₘ-Gruppe, wobei m eine ganze Zahl von 2 bis 12 ist, eine Gruppe mit 2 bis 12 Kohlenstoffatomen, die eine oder mehrere Alkenylgruppen, cis- oder trans- -(CH₂)ₚ-Cyclopropanyl-(CH₂)_{q} enthält, wobei p und q gleiche oder verschieden und ganze Zahlen von 1 bis 4 sind.

9. Verfahren nach einem der vorherigen Ansprüche, wobei R' eine Gruppe ist, die eine fluoreszierende Gruppe oder eine Gruppe umfaßt, an der eine fluroresezierende Gruppe angelagert sein kann.

10. Verfahren nach Anspruch 9, bei dem R' eine Alkoholseitengruppe zur Anlagerung einer fluroeszierenden Gruppe aufweist.

11. Verfahren nach einem der vorherigen Ansprüche, bei dem in Formel (1) X ausgewählt wird aus Hydroxyl-, Carboxyl-, Thiol- und Amingruppen.

12. Verfahren nach Anspruch 11, bei dem X eine Hydroxylgruppe ist.

13. Verfahren nach Anspruch 12, bei dem die Verbindung der Formel (1) eine Verbindung der Formel NC₅R₄-(CH₂)ₙ-OH ist.

14. Verfahren nach einem der vorherigen Ansprüche, bei dem das feste Trägermaterial ein organisches Harz mit einer Funktionalität umfaßt, die mit der Gruppe X der Verbindung der Formel (1) reagieren kann.

15. Verfahren nach Anspruch 14, bei dem das feste Trägermaterial Tritylchlorid oder ein funktionalisiertes Polystyrolharz umfaßt.

16. Verfahren nach einem der vorherigen Ansprüche, bei dem die Gruppe Y in Formel (2) ein Sauerstoffatom ist.

17. Verfahren nach einem der vorherigen Ansprüche, bei dem in Schritt (b) die Gruppe X in einem Mesyl- (Methansulfonyl-)gruppe durch Reaktion mit Mesylchlorid umgewandelt wird.

18. Verfahren nach einem der vorherigen Ansprüche, bei dem A Sauerstoff oder BH₃⁻ ist.

19. Verfahren nach Anspruch 18, bei dem A Sauerstoff ist und das Stickstoffatom der zweiten Pyridinverbindung der Formel (1), die in Schritt (b) verwendet wird, in das N-Oxid durch Reaktion des Stickstoffatoms der Pyridingruppe mit einer Persäure, umgewandelt wird.

20. Verfahren nach Anspruch 19, bei dem die Persäure m-Chlorperbenzoesäure umfaßt.

21. Verfahren nach einem der vorherigen Ansprüche, bei dem das Gegenion Q⁻ in Schritt (b) ein Iodidion ist.

22. Verfahren nach einem der vorherigen Ansprüche, bei dem Oligomere mit der Formel (5) aus dem festen Träger freigesetzt und wieder als Reagenzien als Alternative zu oder zusätzlich zu der zweiten Pyridinverbindung eingeführt werden, die in Schritt (c) verwendet wird.

23. Verfahren nach Anspruch 22, bei dem eine Verbindung der Formel NC₅R₄-R'-[⁻Q⁺NC₅R₄-R'-]ₙ-X (5) in eine Verbindung der Formel ⁻A-NC₅R₄-R'-[⁻Q⁺NC₅R₄-R'-]ₙ-Z (5a) pro Schritt (b) umgewandelt wird, und die Verbindung der Formel (5a) dann mit der Verbindung der Formel (2), die in Schritt (a) oder (c) gebildet wird, pro Schritt (d) reagiert.

24. Verfahren nach einem der vorherigen Ansprüche, bei dem die Verbindung der Formel (4) von dem festen Träger gelöst und reduziert wird, um ein zweifach substituierte Pyridiniumverbindung der Formel NC₅R₄-R'-[⁻O⁺NC₅R₄-R'-]ₙ-X (5) unter Verwendung einer Säure zu bilden.

25. Verfahren nach Anspruch 24, bei dem die Säure Salzsäure und das Gegenion Q⁻ Chlor ist.

26. Verfahren nach einem der vorherigen Ansprüche, bei dem die zweifach substituierte zweifach subsituierte Pyridiniumverbindung mit einer Dialkylpyridiniumverbindung verbunden ist.

## Revendications

1. - Procédé de fabrication d'un composé de pyridinium di-substitué linéaire, le procédé comprenant les étapes consistant à :
(a) fixer un premier composé de pyridine 2, 3 ou 4-substituée de la formule NC₅R₄-R'-X (1) sur un support solide, pour former un composé de la formule NC₅R₄-R' - Y-SUPPORT (2), dans laquelle SUPPORT représente le support solide, R est choisi parmi hydrogène, hydroxyle et les groupes alkyle, alcoxy, aryle, alkaryle, aralkyle et alcényle substitués ou non substitués, R' est un premier groupe de liaison, X est un groupe qui peut réagir avec le support solide pour fixer le premier composé de pyridine au support, et Y est absent ou est un second groupe de liaison ;
(b) former un composé de pyridine di-substituée de la formule ⁻A-⁺NC₅R₄-R'-Z (3) à partir d'un second composé de pyridine 2, 3 ou 4-substituée de formule (1), lequel second composé de pyridine peut être identique ou différent du premier composé de pyridine, A étant un groupe protecteur et Z étant un groupe partant ;
(c) faire réagir le composé de formule (2) formé à l'étape (a) avec le composé de formule (3) formé à l'étape (b), pour former un composé de pyridinium di-substitué de la formule ⁻A-⁺NC₅R₄-R'-[⁻Q⁺NC₅R₄-R'-]ₙY-SUPPORT (4), dans laquelle Q⁻ est un contre-ion et n=1 ;
(d) facultativement, répéter l'étape (c) autant de fois que nécessaire pour obtenir un composé de formule (4) dans laquelle n est un entier de 2 ou plus ; et
(e) détacher le composé de formule (4) du support solide, et le réduire pour former un composé de pyridinium di-substitué de la formule NC₅R₄-R'-[⁻Q⁺NC₅R₄-R'-]ₙ-X (5), dans laquelle n est un entier, et Q⁻ et X sont respectivement un contre-ion et un groupe qui peut réagir avec le support solide pour fixer le premier composé de pyridine au support, qui peuvent être identiques ou différents de Q et X définis ci-dessus dans les étapes respectivement (c) et (a).

2. - Procédé selon la revendication 1, dans lequel chaque groupe R est un hydrogène.

3. - Procédé selon l'une des revendications 1 ou 2, dans lequel, à l'étape (d), l'étape (c) est répétée de telle sorte que, dans la formule (5), n = 20 à 100.

4. - Procédé selon l'une des revendications 1, 2 ou 3, dans lequel le composé de formule (1) est préparé par la réaction d'un composé de formule Z'-R"-X avec un composé de pyridine, avec la protection du groupe X-si nécessaire, R" étant un groupe de liaison et Z' étant un groupe partant approprié.

5. - Procédé selon la revendication 4, dans lequel le composé de formule (1) est préparé par la réaction de Br-R"-OH avec du t-butyldiméthyl-chlorosilane (TBDMSC1) pour former Br-R"-OTBDMS, qui est mis à réagir avec la 2, 3 ou 4-méthylpyridine (2, 3 ou 4-picoline) avec la déprotection du groupe X pour former NC₅R₄-R'-OH.

6. - Procédé selon l'une quelconque des revendications 1 à 5, dans lequel le groupe de liaison R' n'a pas d'atomes de carbone terminaux.

7. - Procédé selon l'une quelconque des revendications précédentes, dans lequel les groupes R' sont identiques ou différents et sont choisis parmi un groupe alkylène, un groupe contenant un alcényle et un groupe contenant un cyclopropanyle.

8. - Procédé selon la revendication 7, dans lequel les groupes R' sont identiques ou différents et sont choisis parmi un groupe -(CH₂)ₘ-, où m est un entier de 2 à 12, un groupe ayant de 2 à 12 atomes de carbone contenant un ou plusieurs groupes alcényle, et cis- ou trans-(CH₂)ₚ-cyclopropanyl- (CH₂)_{q}-, où p et q sont identiques ou différents et sont des entiers de 1 à 4.

9. - Procédé selon l'une quelconque des revendications précédentes, dans lequel R' est un groupe qui comprend un groupe fluorescent ou un groupe auquel un groupe fluorescent peut être attaché.

10. - Procédé selon la revendication 9, dans lequel R' a un groupe alcool pendant, pour la fixation d'un groupe fluorescent.

11. - Procédé selon l'une quelconque des revendications précédentes, dans lequel, dans la formule (1), X est choisi parmi les groupes hydroxyle, carboxyle, thiol et amine.

12. - Procédé selon la revendication 11, dans lequel X est un groupe hydroxyle.

13. - Procédé selon la revendication 12, dans lequel le composé de formule (1) est un composé de la formule NC₅R₄-(CH₂)ₙ-OH.

14. - Procédé selon l'une quelconque des revendications précédentes, dans lequel la matière de support solide comprend une résine organique ayant une fonctionnalité qui peut réagir avec le groupe X du composé de formule (1).

15. - Procédé selon la revendication 14, dans lequel la matière de support solide comprend du chlorure de trityle ou une résine de polystyrène fonctionnalisée.

16. - Procédé selon l'une quelconque des revendications précédentes, dans lequel le groupe Y dans la formule (2) est un atome d'oxygène.

17. - Procédé selon l'une quelconque des revendications précédentes, dans lequel, à l'étape (b), le groupe X est converti en un groupe mésyle (méthanesulfonyle) par réaction avec le chlorure de mésyle.

18. - Procédé selon l'une quelconque des revendications précédentes, dans lequel A est un oxygène ou BH₃- .

19. - Procédé selon la revendication 18, dans lequel A est un oxygène, et l'atome d'azote du second composé de pyridine de formule (1) utilisé à l'étape (b) est converti en le N-oxyde par la réaction de l'atome d'azote du groupe pyridine avec un peracide.

20. - Procédé selon la revendication 19, dans lequel le peracide comprend l'acide m-chloroperbenzoïque.

21. - Procédé selon l'une quelconque des revendications précédentes, dans lequel le contre-ion Q⁻ à l'étape (c) est un ion iodure.

22. - Procédé selon l'une quelconque des revendications précédentes, dans lequel des oligomères ayant la formule, (5) sont libérés du support solide et réintroduits en tant que réactifs comme alternative ou, ou en plus du, second composé de pyridine utilisé à l'étape (b).

23. - Procédé selon la revendication 22, dans lequel un composé de formule NC₅R₄-R'-[⁻Q⁺NC₅R₄-R'-]ₙ-X (5) est converti en un composé de formule ⁻A-⁺NC₅R₄-R'-[⁻Q⁺NC₅R₄-R'-]ₙ-Z (5a) par étape (b), et le composé de formule (5a) est ensuite mis à réagir avec le composé de formule (2) formé à l'étape (a) ou (c), par étape (d).

24. - Procédé selon l'une quelconque des revendications précédentes, dans lequel le composé de formule (4) est détaché du support solide, et réduit pour former un composé de pyridinium di-substitué de la formule NC₅R₄-R'-[⁻Q⁺NC₅R₄-R'-]ₙ-X (5) à l'aide d'un acide.

25. - Procédé selon la revendication 24, dans lequel l'acide est l'acide chlorhydrique, et le contre-ion Q⁻ est le chlorure.

26. - Procédé selon l'une quelconque des revendications précédentes, dans lequel le composé pyridinium di-substitué est un composé de dialkyl pyridinium lié.
